# EUROPEAN PATENT SPECIFICATION

(11) **EP 2 988 735 B1**
(45) Date of publication and mention of the grant of the patent: **24.06.2020**
(21) Application number: 14718330.5
(22) Date of filing: 17.04.2014
(51) Int. Cl.: A61K 9/48, A61K 9/28, A61K 31/381

(54) **PHARMACEUTICAL COMPOSITION COMPRISING A DUAL REUPTAKE INHIBITOR AND METHOD FOR THE PREPARATION THEREOF**
PHARMAZEUTISCHE ZUSAMMENSETZUNG MIT DUALEM WIEDERAUFNAHMEHEMMER UND VERFAHREN ZUR HERSTELLUNG DAVON
COMPOSITION PHARMACEUTIQUE COMPRENANT UN INHIBITEUR DE RÉABSORPTION DOUBLE ET PROCÉDÉ DE PRÉPARATION CORRESPONDANT

(30) Priority: 23.04.2013 GR 20130100262
(43) Date of publication of application: 02.03.2016
(73) Proprietor: Pharmathen S.A., 15351 Pallini Attikis (GR)
(72) Inventor: KARAVAS, Evangelos, GR-153 51 Pallini Attikis (GR); KOUTRIS, Efthimios, GR-153 51 Pallini Attikis (GR); SAMARA, Vasiliki, GR-153 51 Pallini Attikis (GR); KOUTRI, Ioanna, GR-153 51 Pallini Attikis (GR); KALASKANI, Anastasia, GR-153 51 Pallini Attikis (GR); KAKOURIS, Andreas, GR-153 51 Pallini Attikis (GR)
(86) International application number: PCT/EP2014/001041
(87) International publication number: WO 2014/173516

(56) References cited:
- EP-A1- 2 380 563
- EP-A2- 0 693 282
- WO-A1-2009/092129
- WO-A2-2007/034503
- WO-A2-2008/077939
- WO-A2-2008/129501
- WO-A2-2009/118756
- US-A1- 2006 165 776
- US-A1- 2009 226 517
- US-A1- 2010 040 680

## Description

### TECHNICAL FIELD OF THE INVENTION

The present invention relates to a stable pharmaceutical formulation for oral administration containing a therapeutically effective quantity of Duloxetine or a pharmaceutical acceptable salt thereof and a method for the preparation thereof.

### BACKGROUND OF THE INVENTION

Serotonin-norepinephrine reuptake inhibitors (SNRIs) are a class of antidepressant drugs used in the treatment of major depression and other mood disorders. They are sometimes also used to treat anxiety disorders, obsessive-compulsive disorder (OCD), attention deficit hyperactivity disorder (ADHD), chronic neuropathic pain, fibromyalgia syndrome (FMS), and for the relief of menopausal symptoms.

SNRIs act upon and increase the levels of two neurotransmitters in the brain that are known to play an important role in mood. Specifically, they work by inhibiting the reuptake of the neurotransmitters serotonin and norepinephrine. This results in an increase in the extracellular concentrations of serotonin and norepinephrine and, therefore, an increase in neurotransmission. This can be contrasted with the more widely-used selective serotonin reuptake inhibitors (SSRIs) that act only on serotonin. Clinical studies have proven that SNRIs are more effective than SSRIs.

Duloxetine constitutes one of the most commonly used serotonin-norepinephrine reuptake inhibitors. It is used to treat major depressive disorder, generalized anxiety disorder, diabetic peripheral neuropathic pain and fibromyalgia.

The chemical name of Duloxetine is (+)-(S)-N-methyl-3-(naphthalen-1-yloxy)-3-(thiophen-2-yl)propan-1-amine. The molecular formula is C₁₈H₁₉NOS corresponding to a molecular weight of 297.414. It is a white to almost white powder. Duloxetine HCl is sparingly soluble in water, freely soluble in methanol, soluble in anhydrous ethanol and practically insoluble in hexane.

Due to its chemical structure, Duloxetine is prone to acid-catalyzed hydrolysis, which results in the formation of potentially harmful degradation products and a decrease in the bioavailability of Duloxetine. The drug is also sensitive to water hydrolysis.

EP-B-0693282 discloses an enteric formulation of Duloxetine in the form of enteric pellets wherein the enteric layer comprises hydroxypropylmethylcellulose acetate succinate.

WO-A-2008/019712 discloses a pharmaceutical composition of Duloxetine wherein the core is enveloped by a coating comprising methacrylate polymers.

WO-A-2008/077939 discloses a pharmaceutical pellet composition comprising a core comprising Duloxetine and a separating layer comprising a water soluble inorganic salt in the form of crystals.

WO-A-2009/118756 discloses mini tablets comprising a core of Duloxetine, a seal coating over the core, and an enteric coating over the seal coating. The mini tablets are filled into capsules.

Although each of the patents above represents an attempt to overcome the stability problems associated with pharmaceutical compositions comprising Duloxetine, there still exists a need for a stable pharmaceutical composition which avoids the problems related to the intrinsic nature of Duloxetine.

### SUMMARY OF THE INVENTION

It is, therefore, an object of the present invention to provide a stable oral solid dosage formulation for oral administration containing Duloxetine or a pharmaceutical acceptable salt thereof, as an active ingredient, which overcomes the deficiencies of the prior art and avoids degradation of the drug in acidic conditions providing a uniform and constant rate of release over an extended period of time.

It is another object of the present invention to provide a delayed release pharmaceutical composition for oral administration comprising Duloxetine as an active ingredient, which is bioavailable, with sufficient self-life and good pharmacotechnical properties.

Further object of the present invention is to provide a solid dosage form for oral administration containing Duloxetine or a pharmaceutical acceptable salt thereof, which avoids the disadvantages of formulations comprising chemically unstable active ingredients.

An essential object of the present invention is the selection of a separating layer that allows an efficient stabilization of Duloxetine without interfering with its release from the dosage form.

Another essential object of the present invention is to choose an enteric coating comprising a polymer or other material which prevents premature disintegration of Duloxetine in the acidic environment of the stomach, thus avoiding degradation of Duloxetine and promoting release of the drug in the intestine.

A further approach of the present invention is to provide a delayed release dosage form containing Duloxetine which is manufactured through a fast, simple and cost-effective process.

In accordance with the above, a first embodiment of the invention provides a delayed release pharmaceutical composition for oral administration comprising a core of Duloxetine or a pharmaceutical acceptable salt thereof as the active ingredient, a separating layer over the core wherein the thickness of the separating layer is 150-180 µm and an enteric layer over said separating layer, wherein the pharmaceutical composition is in the form of mini tablets filled into a capsule, and each mini-tablet has a diameter of approximately 4 mm. Another embodiment of the present invention concerns a dry granulation process for the preparation of an oral delayed release solid dosage form, in the form of mini tablets filled into a capsule, comprising Duloxetine or a pharmaceutical acceptable salt thereof as the active ingredient, which comprises:
- weighing the active substance and all excipients and sieving;
- blending the active substance with at least one pharmaceutically inert excipient selected from diluents, binders, compression aids and disintegrants until complete homogeneity;
- adding at least one pharmaceutically inert excipient selected from glidants, lubricants, flavours, water scavengers, colorants and sweeteners and mixing until uniformity;
- compressing the obtained granule into mini-tablets of approximately 4 mm diameter;
- applying a separating layer comprising a water soluble coating agent, wherein the thickness of the separating layer is 150-180 µm;
- applying an enteric layer comprising an enteric coating agent over the separating layer; and
- filling capsules with the obtained mini tablets.

Preferred embodiments of the present invention are set out in dependent claims 2 to 5.

Other objects and advantages of the present invention will become apparent to those skilled in the art in view of the following detailed description. The invention is, however, defined by the claims. Any subject-matter falling outside the scope of the claims is provided for information purposes only.

### DETAILED DESCRIPTION OF THE INVENTION

For the purposes of the present invention, a pharmaceutical composition comprising Duloxetine is considered to be "stable" if said ingredient degrades less or more slowly than it does on its own and/or in known pharmaceutical compositions.

As already mentioned the main object of the present invention is to provide a delayed release composition of Duloxetine or a pharmaceutical acceptable salt thereof that is simple to manufacture, bioavailable, cost effective, stable and possesses good pharmacotechnical properties and linearity.

One of the main disadvantages of Duloxetine is the fact that it is susceptible to degradation/hydrolysis and such tendency gets stronger when it is formulated and mixed with excipients. Duloxetine is unstable in solution at pH values less than 2.5; therefore, it degrades in the gastric environment of the stomach. Acid hydrolysis of its ether linkage results in thienyl alcohol and 1-naphthol. Hydrolysis is not desirable as 1-naphtol has toxic properties. Moreover, the manufacturing process of Duloxetine dosage forms should be very carefully selected due to the sensitivity of Duloxetine to water hydrolysis. Accordingly, dry granulation process was finally chosen as the preferred manufacturing process.

The main advantages of the dry granulation process are that less equipment and space is required. Further, said process eliminates the need for binder solution, heavy mixing equipment and time consuming drying step required for wet granulation.

The pharmaceutical composition of the present invention is characterized by physicochemical properties suitable for tablet formulation by direct compression, adequate release rate of the active ingredient (Duloxetine or salts thereof) and storage stability, by employing excipients practically devoiding the tendency to interact with the active ingredient and possessing good compressibility properties.

The present invention's target is to provide a delayed release medicament in the form of a hard gelatin capsule filled with mini tablets. The use of mini tablets is beneficial because pharmacotechnical linearity between the strength and the formulation is easily achieved by incorporating one or more of the mini tablets in the capsule. Linearity is highly desired in the pharmaceutical industry for manufacturing, pharmacokinetic and economical reasons.

Tabletting was the chosen production method because it is faster, easier, adds fewer steps to the process and is the most economical. Further, the tabletting method ensures a high production yield, contrary to the manufacture of pellets where the loss of production output is usually much higher. Furthermore, multi-particulate systems are extremely complex to produce, requiring large numbers of excipients and multiple manufacturing steps. Extrusion-spheronization technique which is the common pellet manufacturing process requires a large quantity of liquid binder to achieve a plastic wet mass. The risk of an accidental high dosage due to possible cracking of coating or not uniform coating of the particulates of multi particular dosage forms is eliminated. Consequently, according to the present invention the finished dosage form is in the form of enteric coated mini-tablets inside a hard gelatin capsule.

Enteric coatings have been used for many years in order to arrest the release of the drug from orally ingestible dosage forms. When such dosage forms are swallowed, they travel down the esophagus to the stomach. In the stomach they are churned and gyrated in highly acidic digestive secretions. If there is anything left of them, it will be passed through the duodenum to the small intestine. The enteric coating protects orally ingestible dosage forms from acidic environments of stomach and allows them to break down easily in pancreatic juices in the duodenum.

However, the use of gastric polymers in the enteric formulation gives rise to several problems regarding the stability of Duloxetine. Since conventionally used gastric coatings contain acidic functional groups, they can cause acid-catalyzed degradation of Duloxetine. Also, it has been shown that Duloxetine reacts with some of these coatings to form amide adducts. These interactions reduce the potency of the drug and can alter the physical characteristics of the coating. In order to eliminate such interactions, the present invention uses a separating layer between the core comprising Duloxetine and the enteric coating to prevent the contact between Duloxetine and the polymers in the enteric coating.

The function of the separating layer is to provide a smooth base for the application of the enteric layer, to prolong resistance to acid conditions, to improve stability by inhibiting any interaction between the drug and the enteric polymer, and finally to improve stability by protecting the drug from light exposure.

The separating layer is preferably composed of a substance (or a mixture of substances) that does not react with the core comprising Duloxetine or a pharmaceutical acceptable salt thereof nor adversely affects bioavailability or release of Duloxetine. Typical examples of such substances that can be used in the separating layer include sugars, celluloses and cellulose derivatives such as hydroxypropylmethyl cellulose, hydroxypropylcellulose, hydroxypropylethyl cellulose, ethylcellulose and the like. The separating layer may be applied onto the core using any known technique. These techniques include for example powder coating, spraying, pan coating and the like.

Water soluble coating agents of the separating layer are present in the preferred composition of the present invention in an amount of more than 5% (w/w) and less than 8% (w/w). Most preferably, they are present in an amount of 6.5% (w/w).

Another important aspect of the separating layer is its thickness that influences product performances in two ways: a) Thick coat improves insulation resulting in improved stability. b) Separating layer minimizes the physical interaction of acidic gastric polymer and the active ingredient, Duloxetine, resulting in complete dissolution of the drug. The thickness of the separating layer is 150-180 µm, more preferably 170 µm

The enteric layer is applied onto the separating layer and comprises enteric materials selected from cellulose acetate phthalate, hydroxypropylmethylcellulose phthalate, hydroxypropylmethylcellulose acetate succinate, polymethacrylates, polivinylacetate phthalate, acrylic acid polymers and the like. The preferred embodiment of the present invention comprises hydroxypropylmethylcellulose acetate succinate, because compatibility studies have shown that it is the most compatible enteric polymer to the drug, Duloxetine. The enteric layer can be applied using one of the conventional spray coating techniques: perforated drum, bottom spray fluid bed coater, top spray fluid bed coater or rotor processor. Enteric coating agents of the enteric layer are present in the preferred composition of the present invention in an amount of more than 8% (w/w) and less than 12% (w/w). Most preferably, they are present in an amount of 10% (w/w).

Both separating and enteric coatings of the pharmaceutical compositions of the present invention may also contain one or more additional ingredients selected from plasticizers, colorants or other excipients to facilitate the formation of a coating.

According to the desired properties of the composition, any number of ingredients may be selected to form the core of the composition, alone or in combination, based upon their known uses in preparation of solid dosage form compositions.

Such ingredients may include, but are not limited to, diluents, binders, compression aids, disintegrants, glidants, lubricants, flavours, water scavengers, colorants and sweeteners. Any optional excipients must be compatible with the active substance so that it does not interfere with it in the composition.

Diluents may be, for example, calcium carbonate, calcium phosphate dibasic, calcium phosphate tribasic, calcium sulfate, microcrystalline cellulose, microcrystalline silicified cellulose, powdered cellulose, dextrates, dextrose, fructose, lactitol, lactose anhydrous, lactose monohydrate, lactose dihydrate, lactose trihydrate, mannitol sorbitol, starch, pregelatinized starch, sucrose, talc, xylitol, mannitol, maltose, maltitol.

Binders may be, for example, acacia mucilage, alginic acid, carbomer, carboxymethylcellulose calcium, carboxymethylcellulose sodium, microcrystalline cellulose, powdered cellulose, ethyl cellulose, gelatin, liquid glucose, guar gum, hydroxyethyl cellulose, hydroxypropyl cellulose, hydroxypropylmethyl cellulose, maltodextrin, methylcellulose, polydextrose, polyethylene oxide, povidone, sodium alginate, starch paste, pregelatinized starch and sucrose.

Disintegrants may be, for example, alginic acid, carbon dioxide, carboxymethylcellulose calcium, carboxymethylcellulose sodium, microcrystalline cellulose, powdered cellulose, croscarmellose sodium, crospovidone, sodium docusate, guar gum, hydroxypropyl cellulose, methylcellulose, polacrilin potassium, poloxamer, povidone, sodium alginate, sodium glycine carbonate, sodium lauryl sulfate, sodium starch glycolate, starch, pregelatinized starch.

Glidants may be, for example, calcium silicate, powdered cellulose, starch, talc and lubricants may be selected from magnesium stearate, polyethylene glycol 4000, polyethylene glycol 6000, sodium lauryl sulfate, starch, talc.

A number of delayed release compositions comprising different excipients were tested as presented in the following examples to achieve the optimal properties with respect to the objectives of the present invention.

### EXAMPLES

**Table 1: Qualitive & Quantitative Compositions 1 to 5**

| **Ingredients** | **1** | **2** | **3** | **4** | **5** |
|---|---|---|---|---|---|
| | **%** | | | | |
| **Internal Phase** | | | | | |
| Duloxetine | 20,00 | 20,00 | 20,00 | 20,00 | 20,00 |
| Duloxetine HCl | 22,44 | 22,44 | 22,44 | 22,44 | 22,44 |
| Mannitol | 21,71 | - | - | - | - |
| Lactose | - | 21,71 | - | - | - |
| Starch 1500 | - | - | 21,71 | 21,71 | 29,45 |
| Microcrystalline cellulose (MCC) | 50,35 | 50,35 | 50,35 | 50,35 | 38,10 |
| Hydroxypropyl cellulose (HPC) | - | - | 4,50 | - | - |
| Polyvinylpyrrolidone (PVP) | 4,50 | 4,50 | - | 4,50 | 4,50 |

| **External Phase** | | | | | |
|---|---|---|---|---|---|
| Talc | 1,00 | 1,00 | 1,00 | 1,00 | 1,50 |
| Magnesium stearate | - | - | - | - | 1,00 |
| Aerosil | - | - | - | - | 1,50 |
| Sodium stearyl fumarate | - | - | - | - | 1,50 |
| **Total** | 100,00 | 100,00 | 100,00 | 100,00 | 100,00 |

| **Separating Coating** | | | | | |
|---|---|---|---|---|---|
| Opadry II White OY-LS 28908 | 6,00 | 6,00 | 6,00 | 6,00 | 6,50 |
| **Enteric coating** | | | | | |
| HPMC Acetate Succinate | 10,00 | 10,00 | 10,00 | 10,00 | 10,00 |

The manufacturing process for the preparation of each of compositions 1 to 5 comprises the following stages:
- The materials that are present in both internal and external phases are weighed and sieved;
- The ingredients of the internal phase are mixed until complete uniformity of the powder;
- In the mixture resulted from the previous step external phase excipients are added and mixed until complete uniformity;
- The granule obtained is compressed into tablets of approximately 4 mm diameter;
- The separating layer is applied onto the core of mini tablets;
- The enteric layer is applied over the separating layer;
- Hard gelatin capsules are filled with the mini tablets.

A direct compression manufacturing process was followed for all the tested formulations (Composition 1 to 5) due to the sensitivity of Duloxetine to water hydrolysis.

Compositions 1 to 3 differ from 4 and 5 in the excipients forming the core of mini tablets. More specifically, composition 1 comprises mannitol and composition 2 lactose instead of starch contained in compositions 4 and 5, while composition 3 comprises hydroxypropylcellulose instead of polyvinylpyrrolidone. Furthermore, the external phase of composition 5 comprises apart from talc also magnesium stearate, aerosil and sodium stearyl fumarate. The thickness of the separating layer was the same for all formulations (approximately 170 µm), while for composition 4 the thickness of the separating layer was different (approximately 60 µm). The composition 4 is not according to the invention.

The five compositions were tested for the active substance release at 100 rpm, dissolution media switch at two hours from buffer with pH value 1.2 to 6.8. In the table 2 below, compositions 1 to 3 and 5 demonstrated satisfactory dissolution rates. However, composition 4 did not provide acceptable results. This is attributed to the thickness of the separating layer.

**Table 2: Comparative dissolution results of Compositions 1 to 5 (Comp. 1 to 5)**

| **TIME (minutes)** | **Comp.1** | **Comp.2** | **Comp.3** | **Comp.4** | **Comp.5** |
|---|---|---|---|---|---|
| 0 | 0 | 0 | 0 | 0 | 0 |
| 60 | 0 | 0 | 0 | 0 | 0 |
| 120 | 1,4% | 0,4% | 0,9% | 1,7% | 0,4% |
| 135 | 8,9% | 36,3% | 58,9% | 12,4% | 54,1% |
| 150 | 67,5% | 95,3% | 94,3% | 30,1% | 95,8% |
| 165 | 80,1% | 99,2% | 96,3% | 44,9% | 96,8% |
| 180 | 87,7% | 99,4% | 97,8% | 57,3% | 97,1% |
| 210 | 98,0% | 98,5% | 98,0% | 73,3% | 98,0% |

Compositions 1 to 3 and 5 were tested for their physical characteristics and feasibility of large scale production. Composition 5 was finally selected as the preferred formulation as the mini-tablets showed better physical characteristics such as flow properties of the bulk and hardness in comparison to compositions 1 to 3.

Finally, composition 5 was tested with respect to the most important object of the present invention that is to prepare a product with acceptable stability. For this reason, capsules of composition 5 were exposed to normal and intermediate conditions (Table 3) for six months. At normal (25°C / RH: 60%) and intermediate conditions (30°C / RH: 65%) the product appeared chemically and physically stable. There was no significant change in assay of active substance, as well as any other tested parameter.

**Table 3: Stability data of composition 5, at 6 months in normal & intermediate conditions**

| | | **25°C/RH 60%** | **30°C/RH 65%** |
|---|---|---|---|
| **Impurities** | **LIMIT** | | |
| IMP B | *NMT 0,20%* | 0,05 | 0,04 |
| IMP C | *NMT 0,20%* | 0,14 | 0,14 |
| IMP E | *NMT 0,20%* | ND | 0,05 |
| IMP F | *NMT 0,10%* | 0,01 | 0,01 |
| IMP D | *NMT 0,20%* | 0,01 | 0,01 |
| IMP G | *NMT 0,10%* | ND | ND |
| TOTAL UNKNOWN | *NMT 0,50%* | 0,16 | 0,22 |
| **TOTAL** | **NMT 1.5%** | **0,37** | **0,47** |

## Claims

1. A delayed release pharmaceutical composition for oral administration comprising a core of Duloxetine or a pharmaceutical acceptable salt thereof as the active ingredient, a separating layer over the core wherein the thickness of the separating layer is 150-180 µm and an enteric layer over said separating layer, wherein the pharmaceutical composition is in the form of mini tablets filled into a capsule, and each mini-tablet has a diameter of approximately 4 mm.

2. The pharmaceutical composition according to claim 1, wherein the separating layer comprises a water soluble coating agent selected from sugars, celluloses and cellulose derivatives such as hydroxypropylmethyl cellulose, hydroxypropylcellulose, hydroxypropylethyl cellulose, ethylcellulose and combinations thereof.

3. The pharmaceutical composition according to claim 1, wherein the enteric layer comprises an enteric coating agent selected from cellulose acetate phthalate, hydroxypropylmethylcellulose phthalate, hydroxypropylmethylcellulose acetate succinate, polymethacrylates, polyvinyl acetate phthalate, acrylic acid polymers and combinations thereof.

4. The pharmaceutical composition according to any preceding claim, wherein the separating and/or enteric layer comprises at least one further excipient selected from plasticizers and/or colorants.

5. The pharmaceutical composition according to any preceding claim, wherein the core comprises at least one pharmaceutically inert excipient selected from diluents, binders, compression aids, disintegrants, glidants, lubricants, flavours, water scavengers, colorants and sweeteners.

6. A dry granulation process for the preparation of an oral delayed release solid dosage form, in the form of mini tablets filled into a capsule, comprising Duloxetine or a pharmaceutical acceptable salt thereof as the active ingredient, which comprises:
- weighing the active substance and all excipients and sieving;
- blending the active substance with at least one pharmaceutically inert excipient selected from diluents, binders, compression aids and disintegrants until complete homogeneity;
- adding at least one pharmaceutically inert excipient selected from glidants, lubricants, flavours, water scavengers, colorants and sweeteners and mixing until uniformity;
- compressing the obtained granule into mini-tablets of approximately 4 mm diameter;
- applying a separating layer comprising a water soluble coating agent, wherein the thickness of the separating layer is 150-180 µm;
- applying an enteric layer comprising an enteric coating agent over the separating layer; and
- filling capsules with the obtained mini tablets.

## Patentansprüche

1. Pharmazeutische Zusammensetzung mit verzögerter Freisetzung zur oralen Verabreichung, umfassend einen Kern aus Duloxetin oder ein pharmazeutisch verträgliches Salz davon als Wirkstoff, eine Trennschicht über dem Kern, wobei die Dicke der Trennschicht 150 bis 180 µm beträgt, und eine enterische Schicht über der Trennschicht, wobei die pharmazeutische Zusammensetzung in Form von Minitabletten vorliegt, die in eine Kapsel gefüllt sind, und jede Minitablette einen Durchmesser von ungefähr 4 mm hat.

2. Pharmazeutische Zusammensetzung nach Anspruch 1, wobei die Trennschicht ein wasserlösliches Beschichtungsmittel umfasst, ausgewählt aus Zuckern, Cellulosen und Cellulosederivaten wie Hydroxypropylmethylcellulose, Hydroxypropylcellulose, Hydroxypropylethylcellulose, Ethylcellulose und Kombinationen davon.

3. Pharmazeutische Zusammensetzung nach Anspruch 1, wobei die enterische Schicht ein enterisches Beschichtungsmittel umfasst, ausgewählt aus Celluloseacetatphthalat, Hydroxypropylmethylcellulosephthalat, Hydroxypropylmethylcelluloseacetatsuccinat, Polymethacrylaten, Polyvinylacetatphthalat, Acrylsäurepolymeren und Kombinationen davon.

4. Pharmazeutische Zusammensetzung nach einem der vorhergehenden Ansprüche, wobei die Trenn- und/oder enterische Schicht mindestens einen weiteren Hilfsstoff umfasst, der aus Weichmachern und/oder Farbstoffen ausgewählt ist.

5. Pharmazeutische Zusammensetzung nach einem der vorhergehenden Ansprüche, wobei der Kern mindestens einen pharmazeutisch inerten Hilfsstoff umfasst, ausgewählt aus Verdünnungsmitteln, Bindemitteln, Kompressionshilfsmitteln, Sprengmitteln, Gleitmitteln, Schmiermitteln, Aromen, Wasserfängern, Farbstoffen und Süßungsmitteln.

6. Trockengranulationsverfahren zur Herstellung einer oralen festen Dosierungsform mit verzögerter Freisetzung in Form von Minitabletten, die in eine Kapsel gefüllt sind und Duloxetin oder ein pharmazeutisch verträgliches Salz davon als Wirkstoff umfassen, umfassend:
- Wiegen des Wirkstoffs und aller Hilfsstoffe und Sieben;
- Mischen des Wirkstoffs mit mindestens einem pharmazeutisch inerten Hilfsstoff, ausgewählt aus Verdünnungsmitteln, Bindemitteln, Kompressionshilfsmitteln und Sprengmitteln, bis zur vollständigen Homogenität;
- Zugabe von mindestens einem pharmazeutisch inerten Hilfsstoff, ausgewählt aus Gleitmitteln, Schmiermitteln, Aromen, Wasserfängern, Farbstoffen und Süßungsmitteln, und Mischen bis zur Gleichmäßigkeit;
- Komprimieren des erhaltenen Granulats zu Minitabletten mit einem Durchmesser von ungefähr 4 mm.
- Aufbringen einer Trennschicht, umfassend ein wasserlösliches Beschichtungsmittel, wobei die Dicke der Trennschicht 150-180 µm beträgt;
- Aufbringen einer enterischen Schicht, die ein enterisches Beschichtungsmittel umfasst, auf die Trennschicht; und
- Kapseln füllen mit den erhaltenen Minitabletten.

## Revendications

1. Une composition pharmaceutique à libération retardée pour administration orale comprenant un noyau de Duloxétine ou un sel pharmaceutiquement acceptable de celle-ci comme principe actif, une couche de séparation sur le noyau d'une épaisseur de 150-180 µm et une couche entérique sur ladite couche de séparation, et où la composition pharmaceutique est sous forme de mini comprimés conditionnés dans une capsule, et chaque mini comprimé a un diamètre d'environ 4 mm.

2. La composition pharmaceutique selon la revendication 1, dans laquelle la couche de séparation comprend un agent d'enrobage soluble dans l'eau sélectionné parmi des sucres, des celluloses et des dérivés de cellulose tels que l'hydroxypropylméthyl cellulose, l'hydroxypropyl cellulose, l'hydroxypropyléthyl cellulose, l'éthyle cellulose et combinaisons de ceux-ci.

3. La composition pharmaceutique selon la revendication 1, dans laquelle la couche entérique comprend un agent d'enrobage entérique sélectionné parmi le phthalate d'acétate de cellulose, le phthalate d'hydroxypropylméthyl cellulose, le succinate d'acétate d'hydroxypropylméthyl cellulose, les poly méthacrylates, le phthalate d'acétate de polyvinyle, les polymères d'acide acrylique et combinaisons de ceux-ci.

4. La composition pharmaceutique selon l'une quelconque des revendications précédentes, dans laquelle la couche de séparation et/ou entérique comprend en outre un autre excipient sélectionné parmi des plastifiants et/ou colorants.

5. La composition pharmaceutique selon l'une quelconque des revendications précédentes, dans laquelle le noyau comprend au moins un excipient pharmaceutiquement inerte sélectionné parmi des diluants, des liants, des auxiliaires de compression, des délitants, des glissants, des lubrifiants, des arômes, des capteurs d'eau, des colorants et des édulcorants.

6. Un procédé de granulation par voie sèche pour la préparation d'une forme posologique à libération retardée pour administration orale, sous forme de mini comprimés conditionnés dans une capsule, comprenant de la Duloxétine ou un sel pharmaceutiquement acceptable de celle-ci comme principe actif, procédé qui comprend:
- peser le principe actif et tous les excipients et tamiser;
- mélanger le principe actif avec au moins un excipient pharmaceutiquement inerte sélectionné parmi des diluants, des liants, des auxiliaires de compression et des délitants jusqu'à homogénéité complète;
- ajouter au moins un excipient pharmaceutiquement inerte sélectionné parmi des glissants, des lubrifiants, des arômes, des capteurs d'eau, des colorants et des édulcorants et mélanger jusqu'à uniformité;
- comprimer le granule obtenu sous forme de mini-comprimés d'environ 4 mm de diamètre ;
- appliquer une couche de séparation comprenant un agent d'enrobage soluble dans l'eau, où l'épaisseur de la couche de séparation est de 150-180 µm;
- appliquer une couche entérique comprenant un agent d'enrobage entérique sur la couche de séparation; et
- remplir des capsules avec les mini-comprimés obtenus.
